# FASCICULE DE BREVET EUROPEEN

(11) **EP 1 800 661 B1**
(45) Date de publication et mention de la délivrance du brevet: **08.07.2009**
(21) Numéro de dépôt: 06301250.4
(22) Date de dépôt: 14.12.2006
(51) Int. Cl.: A61K 8/891, A61K 8/41, A61Q 5/02

(54) **Composition cosmétique comprenant un polymère superabsorbant**
Kosmetische Reinigungsmittel enthaltend ein starkabsorbierendes Polymer
Cosmetic cleaning composition comprising a super-absorbing polymer

(30) Priorité: 22.12.2005 FR 0554034
(43) Date de publication de la demande: 27.06.2007
(73) Titulaire: L'ORÉAL, 75008 Paris (FR)
(72) Inventeur: Parris, Eric, Clichy, 92110 (FR); Samain, Henri, Bievres, 91570 (FR)
(74) Mandataire: Le Coupanec, Pascale A.M.P.

(56) Documents cités:
- WO-A-03/061607
- WO-A1-99/37624
- FR-A- 2 838 335
- US-A1- 2002 061 321
- US-A1- 2003 035 783
- US-B1- 6 482 344
- ANONYMOUS: "Superabsorbent fibre applications" RESEARCH DISCLOSURE, MASON PUBLICATIONS, HAMPSHIRE, GB, vol. 333, no. 66, janvier 1992 (1992-01), XP007117256 ISSN: 0374-4353

## Description

La présente invention concerne de nouvelles compositions cosmétiques destinées plus particulièrement au nettoyage et/ou lavage des matières kératiniques notamment des cheveux.

Pour le nettoyage et/ou le lavage des cheveux, l'utilisation de compositions capillaires détergentes (shampooings), à base essentiellement d'agents tensioactifs classiques de type notamment anionique, non ionique et/ou amphotère, mais plus particulièrement de type anionique, est courante.

Ces compositions sont appliquées sur les cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant mais se présentent presque toujours sous la forme d'un liquide de viscosité modérée.

La présente invention vise plus particulièrement à proposer une composition lavante dotée d'une texture originale comparativement à celle manifestée par les shampooings conventionnels.

Les inventeurs ont ainsi découvert de manière surprenante que l'utilisation de fibres de polymère(s) aux propriétés absorbantes dans des compositions de shampooings permettait d'accéder à des compositions de viscosité très élevée et dotées par ailleurs d'un aspect fibreux ou cotonneux particulièrement original au regard des produits cosmétiques classiques et en particulier dans le domaine des shampooings. Il a été d'autre part observé que de telles compositions conservaient avantageusement les propriétés lavantes des shampooings classiques.

Les polymères dotés de propriétés absorbantes sont déjà utilisés notamment à des fins d'épaississement de produits cosmétiques.

Par exemple, la demande de brevet US 2003/0035783 décrit des gels de coiffage texturés par des polymères aux propriétés absorbantes.

Le document EP 1 195 157 divulgue quant à lui des compositions de produit de maquillage de la peau et des cheveux.

Cependant, dans ces documents, le polymère n'est pas à l'état fibreux et ne confère pas aux compositions correspondantes l'aspect fibreux recherché dans la présente invention. De plus, ces compositions ne contiennent pas de détergent et ne sont pas dédiées au lavage et/ou nettoyage de matières kératiniques.

Pour sa part, la demande internationale de brevet WO 03/061607 concerne des compositions, notamment de shampooing, contenant des biopolymères sous forme de fibres gélifiées à l'image des polysaccharides, comme par exemple les alginates, la gélatine et les caraghenanes. Il est à noter que ces polymères sont dénués de propriétés super absorbantes et ne sont donc pas aptes à conférer aux compositions qui les contiennent la texture fibreuse obtenue par la présente invention.

Ainsi, la présente invention concerne selon un premier aspect une composition de lavage, notamment des matières kératiniques comprenant, dans un milieu physiologiquement acceptable, au moins :
- un polymère superabsorbant présent dans la composition sous forme de fibres, et
- un tensioactif détergent anionique, non ionique ou amphotère.

Selon un autre aspect, l'invention concerne l'utilisation de fibres d'un polymère superabsorbant pour la préparation d'une composition de lavage des fibres kératiniques, notamment un shampooing.

La présente invention concerne également un procédé de traitement cosmétique des matières kératiniques qui comprend l'application d'une quantité efficace d'une composition telle que décrite ci-dessus sur les matières kératiniques et la réalisation d'un rinçage après un éventuel temps de pose.

Les fibres insolubles de polymère superabsorbant considéré selon l'invention peuvent être reparties de manière uniforme ou non dans la composition. Elles sont généralement en quantité suffisante pour conférer à la composition un épaississement significatif reflété par une augmentation importante de la viscosité au niveau de la composition considérée.

Plus précisément, cette viscosité à 25 °C est de préférence supérieure à 1 Pa.s, en particulier varie de 1 Pa.s à 1.000 Pa.s, et notamment de 10 à 500 Pa.s, et encore plus préférentiellement de 30 à 500 Pa.s à un taux de cisaillement de 1s⁻¹. Elle est appréciée de préference à l'aide d'un viscosimètre, comme le viscosimètre HAAKE RS1 de THERMOELECTRON (viscosimètre à géométrie cône plan).

Par "physiologiquement acceptable", on entend un milieu compatible avec l'application sur le corps d'un être vivant, notamment d'un être humain, en particulier sur sa matière kératinique, notamment les cheveux.

Dans l'ensemble de la description, les expressions "compris entre ... et ...", "variant de ... à ..." et "allant de ... à ..." sont à comprendre dans un sens large englobant les bornes.

Outre une texture très originale et des propriétés lavantes satisfaisantes, ces compositions présentent d'excellentes qualités de démarrage, d'abondance et de volume de mousse.

D'autre part, la viscosité élevée des compositions permet d'éviter les coulures à l'application. Les compositions selon l'invention peuvent être utilisées dans une moindre quantité pour une même abondance de mousse par rapport aux shampooings classiques.

Enfin, la texture pâteuse du produit permet une consommation au plus juste.

### POLYMERE SUPERABSORBANT

Selon la présente invention, on entend par "polymère superabsorbant", un polymère apte, lorsqu'il est à l'état sec, à absorber spontanément au moins 20 fois son poids de fluide aqueux, en particulier d'eau et notamment d'eau distillée. De tels polymères super absorbants sont décrits dans l'ouvrage "Absorbent polymer technology, Studies in polymer science 8" de L. BRANNON-PAPPAS et R. HARLAND, édition Elsevier, 1990.

Ces polymères ont une grande capacité d'absorption et de rétention de l'eau et de fluides aqueux. Après absorption du liquide aqueux, les particules du polymère ainsi imbibées de fluide aqueux restent insolubles dans le fluide aqueux et y conservent leur état particulaire individualisé.

Ainsi, le polymère superabsorbant peut posséder à l'état sec une capacité d'absorption d'eau allant de 20 à 2000 fois son propre poids (soit 20 g à 2000 g d'eau absorbée par gramme de polymère absorbant), de préférence de 30 à 1500 fois, et mieux de 50 à 1000 fois. Ces caractéristiques d'absorption d'eau sont définies aux conditions normales de température (25 °C) et de pression (760 mm Hg soit 100 000 Pa) et pour de l'eau distillée.

La valeur de la capacité d'absorption d'eau d'un polymère peut être déterminée en dispersant 0,5 g de polymère(s) dans 150 g d'une solution d'eau, en attendant 20 minutes, en filtrant la solution non absorbée sur un filtre de 150 µm pendant 20 minutes et en pesant l'eau non absorbée.

Les fibres de polymère superabsorbant ont généralement, à l'état sec, une longueur moyenne en nombre supérieure à 0,5 mm, de préférence supérieure ou égale à 1 mm, notamment allant de 1 mm à 100 mm, en particulier variant de 2 à 80 mm, voire de 3 à 60 mm, et mieux variant de 3 mm à 10 mm. On entend par longueur des fibres la plus grande dimension de ces fibres.

A l'état sec, les fibres ont généralement une largeur moyenne en nombre allant de 1 à 100 µm, en particulier de 5 à 80 µm, encore mieux de 10 à 60 µm, par exemple une largeur d'environ 30 µm. La largeur peut correspondre au diamètre de la fibre, notamment lorsque la fibre a une section sensiblement circulaire.

Ainsi, à l'état sec, les fibres de polymère superabsorbant peuvent avoir un rapport d'aspect (longueur/largeur) allant de 50 à 10 000, de préférence de 50 à 2500, plus préférentiellement de 75 à 1000 et encore mieux de 100 à 500, par exemple environ 200.

En présence d'eau, ces fibres gonflent.

Le polymère superabsorbant peut être partiellement ou totalement gonflé dans la composition selon l'invention.

Le polymère absorbant peut être introduit dans la composition sous forme sèche. Le polymère peut également être introduit dans un état partiellement gonflé. Pour ce faire, le polymère superabsorbant peut être mis à gonfler dans une partie du solvant, notamment de l'eau, de la composition avant d'être introduit dans la composition.

Dans la composition selon l'invention, les fibres ont généralement une largeur moyenne en nombre allant de 5 à 500 µm, de préférence 25 à 400 µm, encore mieux de 50 à 300 µm et par exemple une largeur d'environ 150 µm.

Ainsi, en passant de l'état sec à l'état mouillé (dans la composition), les fibres peuvent avoir une largeur augmentant d'un facteur d'environ 5.

Dans la composition, les fibres peuvent avoir un rapport d'aspect allant de 1 à 2000, de préférence de 2 à 200, plus préférentiellement de 15 à 150, et encore mieux de 20 à 100 et par exemple d'environ 40.

Ainsi, en passant de l'état sec à l'état mouillé, le rapport d'aspect diminue. En particulier, le rapport (rapport d'aspect à l'état gonflé/rapport d'aspect à l'état sec) peut aller de 4 à 100 %, de préférence de 8 à 75 %, encore mieux de 10 à 50 %, par exemple être d'environ 20 %.

Le polymère superabsorbant est différent d'un biopolymère et, en particulier, est un polymère synthétique. Par polymère synthétique, on entend polymère obtenu chimiquement ou par production dans un organisme des éléments nécessaires à sa production.

Comme exemple de polymères superabsorbants, on peut citer : les polymères résultant de la polymérisation avec réticulation partielle de monomères à insaturation éthylénique hydrosolubles, comme les polymères acryliques ou vinyliques, en particulier les polyacrylates réticulés et partiellement ou totalement neutralisés.

Comme polymère superabsorbant conduisant à des fibres dans les compositions de l'invention, on peut citer les produits référencés OASIS 101 3 mm ou OASIS SUB 250 MICRON ou OASIS 121/6/10 ABSORBENTS proposés par la société TECHNICAL à base d'un copolymère acrylate réticulé partiellement neutralisé et dont les longueurs respectives moyennes des fibres à l'état sec sont de l'ordre de 3 mm, 250 µm et 6 mm.

La composition selon l'invention comprend généralement le(s) polymère(s) superabsorbant(s), et notamment les fibres de polymère superabsorbant, en une teneur exprimée à l'état sec supérieure ou égale à 0,1 % en poids, par rapport à son poids total, et de préférence allant de 1 à 50 % en poids, et mieux de 3 à 40 % en poids, voire de 5 à 25 % en poids. Avantageusement, cette quantité peut également être ajustée pour conférer une texture originale à la composition et en particulier la doter d'un aspect fibreux.

### TENSIOACTIF DETERGENT

Le ou les tensioactifs détergents sont choisis, seuls ou en mélanges, parmi les tensioactifs anioniques, non ioniques et amphotères ou zwittérioniques.

Les tensioactifs convenant à la mise en oeuvre de la présente invention sont notamment les suivants :

### i. Tensioactif(s) anionique(s):

Les compositions selon l'invention peuvent ainsi comprendre au moins un tensioactif anionique.

A titre d'exemple de tensio-actifs anioniques utilisables, seuls ou en mélange, dans le cadre de la présente invention, on peut citer notamment les sels (en particulier sels alcalins, notamment sels de sodium, sels d'ammonium, sels d'amines, sels d'aminoalcools ou sels de magnésium) des composés suivants : les alkylsulfates, alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates; les alkylsulfosuccinates, alkyléthersulfosuccinates, alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle.

Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D-galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (1) suivante:

R₁- (OC₂H₄)ₙ-OCH₂COOA (1)

dans laquelle :
- R₁ désigne un groupement alkyle, alkylamido ou alkaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,
- A désigne H, ammonium, Na, K, Li, Mg ou un reste monoéthanolamine ou triéthanolamine. On peut également utiliser des mélanges de composés de formule (1) en particulier des mélanges dans lesquels les groupements R₁ sont différents.

Des composés de formule (1) sont vendus par exemple par la Société CHEM Y sous les dénominations AKYPO (NP40, NP70, OP40, OP80, RLM25, RLM38, RLMQ 38 NV, RLM 45, RLM 45 NV, RLM 100, RLM 100 NV, RO 20, RO 90, RCS 60, RS 60, RS 100, RO 50) ou par la Société SANDOZ sous les dénominations SANDOPAN (DTC Acid, DTC).

Les tensioactifs anioniques convenant plus particulièrement aux compositions selon l'invention sont choisis parmi le lauryléther sulfate de sodium ou d'ammonium, le lauryl sulfate de sodium ou d'ammonium et/ou leurs mélanges.

La quantité d'agents tensioactifs anioniques varie, de préférence, de 4 % à 50 % en poids, rapportée au poids total de la composition cosmétique. Elle est de préférence comprise entre 5 % et 35 % en poids, et mieux encore, entre 8 % et 25 % en poids.

### ii. Tensioactif(s) non ionique(s):

Les compositions selon l'invention peuvent comprendre au moins un tensioactif non ionique.

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178).

Ainsi, ils peuvent être notamment choisis parmi les alcools gras polyéthoxylés, polypropoxylés ou polyglycérolés, les alpha-diols gras polyéthoxylés, polypropoxylés ou polyglycérolés, les alkylphénols gras polyéthoxylés, polypropoxylés ou polyglycérolés et les acides gras polyéthoxylés, polypropoxylés ou polyglycérolés, tous ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglucosides, les dérivés de N-alkyl glucamine, les oxydes d'amines tels que les oxydes d'alkyl (C₁₀- C₁₄) amines ou les oxydes de N-acylaminopropylmorpholine.

On notera que les alkypolyglucosides (telles que la cocoglucoside) constituent des tensio-actifs non-ioniques convenant tout particulièrement à la présente invention.

La quantité d'agents tensioactifs non ioniques lorsqu'ils sont présents est de préférence comprise entre 0,5 et 20 %, et en particulier entre 1 et 15 % rapportée au poids total de la composition.

### iii. Tensioactif(s) amphotère(s) ou zwittérionique(s):

La composition peut également comprendre un ou plusieurs tensioactif(s) amphotère(s).

Les agents tensioactifs amphotères ou zwitterioniques peuvent être notamment des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; en particulier les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives:

R₂ -CONHCH₂CH₂-N⁺(R₃)(R₄)(CH₂COO⁻) (2)

dans laquelle :
- R₂ désigne le radical alkyle d'un acide R₂-COOH présent dans l'huile de coprah hydrolysée, un radical heptyle, nonyle ou undécyle,
- R₃ désigne un groupement bêta-hydroxyéthyle, et
- R₄ un groupement carboxyméthyle;
et

R₂,-CONHCH₂CH₂-N(B)(C) (3)

dans laquelle:
- B représente -CH₂CH₂OX', X' désignant le groupement -CH₂CH₂-COOH ou un atome d'hydrogène,
- C représente -(CH₂)_{z}-Y', avec z égalant 1 ou 2, Y' désignant -COOH ou le radical -CH₂-CHOH-SO₃H et X' étant tel que défini précédemment, et
- R₂, désigne le radical alkyle d'un acide R₂,-COOH présent dans l'huile de coprah ou dans l'huile de lin hydrolysée, un radical alkyle, notamment en C₇, C₉, C₁₁ ou C₁₃, un radical alkyle en C₁₇ et sa forme iso ou un radical C₁₇ insaturé.

A titre d'exemple on peut citer le cocoamphocarboxyglycinate vendu sous la dénomination commerciale MIRANOL C2M concentré par la Société MIRANOL.

Dans le cadre de l'invention, les tensioactifs amphotères particulièrement préférés sont avantageusement choisis parmi la coco bétaïne, la cocoamidopropylbétaïne ou leurs mélanges.

La quantité et la qualité des tensioactifs sont ajustées pour conférer à la composition finale un pouvoir détergent et/ou moussant satisfaisant.

Ainsi, selon l'invention, les compositions peuvent, de préférence, comprendre 3 % en poids ou plus de tensioactif(s) détergent(s) et notamment peuvent comprendre de 4 % à 50 % en poids, de préférence de 6 % à 25 % en poids, et encore plus préférentiellement de 10 % à 18 % en poids de tensioactif(s) détergent(s). De préférence, les compositions comprennent au moins un tensioactif anionique et au moins un tensioactif amphotène ou non ionique, et de préférence amphotère.

Dans le cadre de l'invention, on peut associer un polymère acrylique, en particulier un sel alcalin de copolymère acrylique réticulé avec un alkyléther sulfate tel que le lauryléther sulfate de sodium, une alkylpolyglucoside telle que la cocoglucoside et/ou une alkylbétaïne telle que la cocobétaïne.

### PARTICULES

Dans un mode de réalisation particulier, les compositions de lavage de l'invention peuvent comprendre en outre des particules insolubles différentes des fibres de polymères absorbants.

La présence de particules insolubles permet notamment de prolonger la conservation dans le temps de l'aspect fibreux des compositions voire d'accroître l'aspect fibreux au niveau de la composition.

Ces particules peuvent être minérales ou organiques.

Les particules organiques peuvent notamment être choisies parmi les particules de verre, de polyamides tels que le Nylon, de polymères ou copolymères d'acrylonitrile, de chlorure de vinylidène, de chlorure de vinyle et/ou de monomère acrylique ou styrénique, éventuellement expansés. Le monomère acrylique est par exemple un acrylate ou méthacrylate de méthyle ou d'éthyle. Le monomère styrénique est par exemple l'α-méthyl-styrène ou le styrène.

Les particules minérales comprennent de préférence au moins un élément des colonnes IIa, IIIa, IVa, IIIb et IVb du tableau de classification périodique des éléments et de préférence des colonnes IIa, IIIa et IVa. Elles sont notamment choisies parmi les particules contenant au moins 10 % en poids de carbonate de calcium ou d'au moins un silicate, les particules contenant au moins 90 % d'oxyde d'aluminium, les silices, l'oxyde de magnésium, les oxydes de Zinc, les oxydes de titane, le sulfate de baryum, le nitrure de Bore et leurs mélanges.

Les silicates utilisables selon l'invention peuvent être choisis parmi les silicates de sodium, de magnésium tel que le talc et/ou de lithium, On peut notamment utiliser les composés commercialisés par la société LAPORTE sous la dénomination LAPONITE XLG et LAPONITE XLS.

Les silicates convenant dans les compositions de la présente invention peuvent être d'origine naturelle ou d'origine synthétique.

Les particules solides présentent, de préférence, une taille primaire moyenne en nombre comprise entre 2 nm et 2 microns, plus préférentiellement entre 5nm et 500 nm, et plus préférentiellement encore entre 10 nm et 250 nm. Au sens de la présente invention, on entend par "taille primaire de particules", la dimension maximale qu'il est possible de mesurer entre deux points diamétralement opposés d'une particule individuelle.

Les particules selon l'invention peuvent, par exemple, avoir une forme quelconque, par exemple la forme de sphères, de paillettes, d'aiguilles, de plaquettes ou des formes totalement aléatoires. De préférence, elles sont sous forme de plaquettes.

Selon l'invention, les compositions peuvent comprendre de 0,001 % à 20 % en poids, de préférence de 0,01 % à 10% en poids et plus particulièrement de 0,1 à 3 % en poids de particules insolubles.

Le rapport pondéral particules/fibres peut varier de 0,2 à 10, en particulier de 0,5 à 1,2.

Selon un mode particulier de l'invention, la composition comprend des fibres de copolymère acrylate réticulé, notamment un polyacrylate de sodium et des plaquettes de talc et/ou de nitrure de Bore.

### MILIEU PHYSIOLOGIQUEMENT ACCEPTABLE

Le milieu physiologiquement acceptable comprend préférentiellement de l'eau.

Le milieu aqueux peut être constitué uniquement par de l'eau ou par un mélange d'eau et d'un solvant cosmétiquement acceptable tel qu'un alcool inférieur en C₁-C₄, tel que l'éthanol, l'isopropanol, le tertio-butanol, le n-butanol ; les polyols comme le propylèneglycol, les éthers de polyol ; et leurs mélanges.

De préférence, la composition comprend plus de 30 %, voire de 50 à 95 % en poids d'eau par rapport au poids total de la composition.

Les compositions de lavage selon l'invention présentent un pH final généralement compris entre 3 et 10. De préférence, ce pH est compris entre 4,5 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

### AUTRES COMPOSES

Les compositions conformes à l'invention peuvent contenir en plus des polymères superabsorbants d'eau, d'autres composés régulateurs de viscosité tels que des électrolytes, ou des agents épaississants (associatifs ou non associatifs). On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination "AMINOL A15" par la société CHEM Y, les acides polyacryliques réticulés et les copolymères d'acide acrylique tels que les copolymères d'acide acrylique et d'acrylates d'alkyle en C₁₀-C₃₀ réticulés, à l'état non fibreux. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir de préférence jusqu'à 5 % d'agents nacrants ou opacifiants bien connus dans l'état de la technique tels que par exemple les alcools gras supérieurs à C₁₆, les dérivés acylés à chaîne grasse tels que les monostéarates ou distéarates d'éthylène glycol ou de polyéthylèneglycol, les éthers à chaînes grasses (C₁₀-C₃₀) tels que par exemple le distéaryléther ou le 1-(hexadécyloxy)-2-octadécanol. 1.

Les compositions conformes à l'invention peuvent éventuellement contenir en outre au moins un additif choisi parmi les synergistes de mousses tels que des 1,2-alcanediols en C₁₀-C₁₈ ou des alcanolamides gras dérivés de mono ou de diéthanolamine, les filtres solaires siliconés ou non, les polymères anioniques ou non ioniques, les protéines, les hydrolysats de protéines, les céramides, les pseudocéramides, les acides gras à chaînes linéaires ou ramifiées en C₁₂-C₄₀ tels que l'acide méthyl-18 eicosanoique, les hydroxyacides, les vitamines, les provitamines tels que le panthénol, les huiles animales, minérales ou de synthèse et tout autre additif classiquement utilisé dans le domaine cosmétique qui n'affecte pas les propriétés des compositions selon l'invention.

Les compositions de lavage selon l'invention peuvent bien entendu contenir en outre tous les adjuvants usuels rencontrés dans le domaine des shampooings, comme par exemple des parfums, des agents conservateurs, des sequestrants, des adoucissants, des colorants, des agents hydratants, des agents anti-pélliculaires ou anti-séborrhéiques, et autres.

Les compositions conformes à l'invention peuvent éventuellement contenir en outre d'autres agents ayant pour effet d'améliorer les propriétés cosmétiques de cheveux ou de la peau sans cependant altérer la stabilité des compositions. On peut citer à ce sujet les polymères anioniques ou non ioniques ou amphotères, les protéines, les hydrolysats de protéines, les cires naturelles ou synthétiques, les céramides, les pseudocéramides, les acides gras à chaînes linéaires ou ramifiées en C₁₆-C₄₀ tels que l'acide méthyl-18 eicosanoique, les esters gras d'acides carboxyliques, les mono-, di- ou triglycérides d'acides gras, les vitamines, les provitamines telles que le panthénol, les huiles végétales, les huiles de synthèses telles que les poly-alphaoléfmes, les huiles fluorées ou perfluorées, et leurs mélanges.

Les compositions selon l'invention comprennent de préférence au moins une silicone.

A titre de silicones utilisables dans les compositions de la présente invention, on peut notamment citer les silicones volatiles ou non, cycliques ou acycliques, ramifiées ou non, organomodifiées ou non, telles que décrites ci-dessous.

Les silicones utilisables conformément à l'invention peuvent être solubles ou insolubles dans la composition et en particulier être des polyorganosiloxanes insolubles dans la composition de l'invention ; elles peuvent se présenter sous forme d'huiles, de cires, de résines ou de gommes.

Selon l'invention, toutes les silicones peuvent être utilisées telle quelle ou sous forme de solutions, de dispersions, d'émulsions, de nanoémulsions ou de microémulsions.

Les organopolysiloxanes sont définis plus en détail dans l'ouvrage de Walter NOLL "Chemistry and Technology of Silicones" (1968) Academie Press. Elles peuvent être volatiles ou non volatiles.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25 °C et de préférence 1.10⁻⁵ à 1 m²/s. La viscosité des silicones est, par exemple, mesurée à 25 °C selon la norme ASTM 445 Appendice C.

Parmi ces polyalkylsiloxanes, on peut citer à titre non limitatif les produits commerciaux suivants :
- les huiles SILBIONE^{®} des séries 47 et 70 047 ou les huiles MIRASIL^{®} commercialisées par RHODIA telles que, par exemple, l'huile 70 047 V 500 000 ;
- les huiles de la série MIRASIL^{®} commercialisées par la société RHODIA ;
- les huiles de la série 200 de la société DOW CORNING telles que plus particulièrement la DC200 de viscosité 60 000 mm²/s;
- les huiles VISCASIL^{®} de GENERAL ELECTRIC et certaines huiles des séries SF (SF 96, SF 18) de GENERAL ELECTRIC.

On peut également citer les polydiméthylsiloxanes à groupements terminaux diméthylsilanol, connus sous le nom de dimethiconol (CTFA), tels que les huiles de la série 48 de la société RHODIA.

Dans cette classe de polyalkylsiloxanes, on peut également citer les produits commercialisés sous les dénominations "ABIL WAX^{®} 9800 et 9801" par la société GOLDSCHMIDT qui sont des polyalkyl(C₁-C₂₀)-siloxanes.

Il peut s'agir de polyalkylarylsiloxanes, notamment choisis parmi les polydiméthyl/méthylphénylsiloxanes, les polydiméthyldiphényl-siloxanes linéaires et/ou ramifiés de viscosité de 1.10⁻⁵ à 5.10⁻² m²/s à 25 °C.

Parmi ces polyalkylarylsiloxanes on peut citer, à titre d'exemple, les produits commercialisés sous les dénominations suivantes :
- les huiles SILBIONE^{®} de la série 70 641 de RHODIA ;
- les huiles des séries RHODORSIL^{®} 70 633 et 763 de RHODIA ;
- l'huile DOW CORNING 556 COSMETIC GRAD FLUID de DOW CORNING ;
- les silicones de la série PK de BAYER comme le produit PK20;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Des produits plus particulièrement utilisables conformément à l'invention sont des mélanges tels que :
- les mélanges formés à partir d'un polydiméthylsiloxane hydroxylé en bout de chaîne ou diméthiconol (CTFA) et d'un poly-diméthylsiloxane cyclique également appelé cyclométhicone (CTFA) tel que le produit Q2 1401 commercialisé par la société DOW CORNING ;
- les mélanges formés à partir d'une gomme polydiméthyl-siloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶ m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
R₂SiO_{2/2}, R₃SiO_{1/2}, RSiO_{3/2} et SiO_{4/2} dans lesquels R représente un groupement hydrocarboné possédant 1 à 16 atomes de carbone ou un groupement phényle. Parmi ces produits, ceux particulièrement préférés sont ceux dans lesquels R désigne un groupe alkyle inférieur en C₁-C₄, plus particulièrement méthyle, ou un groupe phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthylsiloxane.

On peut également citer les résines du type triméthylsiloxysilicate commercialisées notamment sous les dénominations X22-4914, X21-5034 et X21-5037 par la société SHIN-ETSU.

Les silicones telles que décrites ci-dessus peuvent être utilisées seules ou en mélange, en une quantité comprise entre 0,01 et 20 % en poids, de préférence entre 0,1 et 5 % en poids par rapport au poids total de la composition.

La composition selon l'invention peut comprendre en outre un ou plusieurs polymères cationiques. Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863.

Au sens de la présente invention, l'expression "polymère cationique" désigne tout polymère comprenant des groupements cationiques et/ou des groupements ionisables en groupements cationiques.

Les polymères cationiques préférés sont choisis parmi ceux qui contiennent des motifs comportant des groupements amines primaires, secondaires, tertiaires et/ou quaternaires pouvant soit faire partie de la chaîne principale polymère, soit être portés par un substituant latéral directement relié à celle-ci.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français 2 505 348 ou 2 542 997. Parmi ces polymères, on peut citer :
- les copolymères d'acrylamide et de diméthylaminoéthyl méthacrylate quaternisé au sulfate de diméthyle ou avec un halogénure de diméthyle tels que celui vendu sous la dénomination HERCOFLOC par la société HERCULES,
- les copolymères d'acrylamide et de chlorure de méthacryloyloxyéthyl-triméthylammonium décrit par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyl-triméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français FR 2 077 143 et 2 393 573,
- les terpolymères méthacrylate de diméthyl amino éthyle/ vinylcaprolactame/vinylpyrrolidone tel que le produit vendu sous la dénomination GAFFIX VC 713 par la société ISP,
- les copolymères vinylpyrrolidone / méthacrylamidopropyl dimethylamine commercialisés notamment sous la dénomination STYLEZE CC 10 par ISP.
- et les copolymères vinylpyrrolidone / méthacrylamide de diméthylaminopropyle quaternisé tel que le produit vendu sous la dénomination "GAFQUAT HS 100" par la société ISP.

Conviennent également à titre de polymères cationiques, les polysaccharides cationiques notamment les celluloses à l'image par exemple des polymères commercialisés sous les dénominations "JR" (JR 400, JR 125, JR 30M) ou "LR" (LR 400, LR 30M) par la Société AMERCHOL et les produits commercialisés sous la dénomination "Celquat L 200" et "Celquat H 100" par la Société National Starch et les gommes de galactomannanes cationiques comme par exemple les produits commercialisés notamment sous les dénominations commerciales de JAGUAR C13 S, JAGUAR C 15, JAGUAR C 17 ou JAGUAR C162 par la société RHODIA.

Les polymères cationiques convenant à l'invention peuvent également être choisis parmi :
- les dérivés de polyaminoamides comme par exemple les polymères acide adipique/diméthylaminohydroxypropyl/diéthylène triamine vendus sous la dénomination "Cartaretine F, F4 ou F8" par la société Sandoz,
- les polymères dérivant de polyalkylène polyamine comme par exemple ceux commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
- les cyclopolymères d'alkyl diallyl amine ou de dialkyl diallyl ammonium tels que les polymères vendus sous la dénomination "MERQUAT 100" par la société NALCO (et leurs homologues de faibles masses molaires moyenne en poids) et les copolymères de sels (par exemple chlorure) de diallyldiméthylammonium et d'acrylamide par exemple commercialisés sous la dénomination "MERQUAT 550",
- les polymères de diammonium ou de polyammonium quaternaires comme par exemple les produits "Mirapol^{®} A 15", "Mirapol^{®} AD1", "Mirapol^{®} AZ1" et "Mirapol^{®} 175" vendus par la société Miranol,
- les polymères quaternaires de vinylpyrrolidone et de vinylimidazole tels que par exemple les produits commercialisés sous les dénominations Luviquat^{®} FC 905, FC 550 et FC 370 par la société B.A.S.F,
- les polyamines comme le Polyquart^{®} H vendu par COGNIS, référencé sous le nom de "POLYETHYLENEGLYCOL (15) TALLOW POLYAMINE" dans le dictionnaire CTFA, et
- les polymères de préférence réticulés de sels de méthacryloyloxyalkyl(C₁-C₄) trialkyl(C₁-C₄)ammonium tels que par exemple les produits commercialisés à l'état de dispersion sous le nom de "SALCARE^{®} SC 92", de "SALCARE^{®} SC 95" et "SALCARE^{®} SC 96" par la Société CIBA.

D'autres polymères cationiques utilisables dans le cadre de l'invention sont des protéines cationiques ou des hydrolysats de protéines cationiques, des polyalkylèneimines, en particulier des polyéthylèneimines, des polymères comprenant des motifs vinylpyridine ou vinylpyridinium, des condensats de polyamines et d'épichlorhydrine, des polyuréylènes quaternaires et les dérivés de la chitine.

Parmi les polymères cationiques mentionnés ci-dessus, convenant dans l'invention, on peut utiliser de préférence les dérivés d'éther de cellulose quaternaires tels que les produits vendus sous la dénomination "JR 400" par la Société AMERCHOL, les cyclopolymères cationiques, en particulier les homopolymères ou copolymères de sels (par exemple chlorure) de diméthyldiallylammonium, vendus sous les dénominations "MERQUAT 100", "MERQUAT 550" et "MERQUAT S" par la société NALCO et leurs homologues de faibles poids moléculaires en poids, les polymères quaternaires de vinylpyrrolidone et de vinylimidazole, les homopolymères ou copolymères éventuellement réticulés de sels de méthacryloyloxyalkyl(C₁-C₄) trialkyl(C₁-C₄)ammonium et leurs mélanges.

Le ou les polymères cationiques sont généralement présents à des concentrations allant de 0,01 à 20 %, de préférence de 0,05 à 10 % et plus particulièrement de 0,1 à 5 % en poids par rapport au poids total de la composition.

Bien entendu, l'homme de l'art veillera à choisir ce ou ces éventuels composés complémentaires et/ou leurs quantités de manière telle que les propriétés avantageuses attachées intrinsèquement à l'association conforme à l'invention ne soient pas, ou substantiellement pas, altérées par la ou les adjonctions envisagées.

Ces compositions conviennent principalement au lavage des cheveux.

Selon un mode de réalisation préféré de l'invention, la composition peut être utilisée comme shampooing.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

Les compositions selon l'invention peuvent être préparées par mélange des différents constituants à température ambiante et soumission de l'ensemble à un fort cisaillement.

Selon un premier mode de réalisation, l'ensemble des constituants de la composition est mélangé préalablement à l'opération de cisaillement.

Selon un autre mode de réalisation, l'ensemble des composants, à l'exception des fibres, est homogénéisé au préalable, avant ajout des fibres. Il est également possible de procéder au mélange des composés sous chauffage.

Les exemples suivants sont donnés à titre illustratif et non limitatif de la présente invention.

### EXEMPLES

| Nom | Concentration (% massique) | | | | | |
|---|---|---|---|---|---|---|
| | Exemple **1** | Exemple **2** | Exemple **3** | Exemple **4** | Exemple **5** | Exemple **6** |
| Lauryl éther sulfate de sodium | 12 | 15 | 13 | 12 | 13.5 | - |
| Coco bétaïne¹ | 2.4 | 2.4 | 2.4 | 1.8 | 2.4 | - |
| Coco-glucoside² | - | - | - | - | - | 15 |
| Sel de Na de copolymère acrylate réticulé³ | 12 | 9 | 10 | 12 | 8 | 12 |
| Nitrure de Bore⁴ | - | 6 | - | - | 6 | - |
| Talc⁵ | - | - | 10 | 16 | - | 6,5 |
| Glycérine⁶ | - | 10 | 0 | 4.5 | 10 | - |
| Polyquaternium 10⁷ | - | 0,5 | 0,5 | 0,5 | 0,5 | 0,5 |
| Dimethicone laureth-4, laureth-23⁸ | - | - | - | - | 6 | - |
| Conservateurs | qs | qs | qs | qs | qs | - |
| Parfum | qs | qs | qs | qs | qs | - |
| Colorant | qs | qs | qs | qs | qs | - |
| Agent de pH | 7±0.5 | 7±0.5 | 7±0.5 | 7±0.5 | 7±0.5 | - |
| Eau qsp | 100 | 100 | 100 | 100 | 100 | 100 |

| | | | | | | |
|---|---|---|---|---|---|---|
| ¹ : Mirataine BB/FLA - RHODIA ² : Plantacare 818 up - COGNIS ³ : Oasis 121/6/10 - TECHNICAL ABSORBENTS dont les fibres ont longueur moyenne en nombre de 6 mm. ⁴ : Boron nitride powder CC6004 - ADVANCED CERAMICS ⁵ : Rose Talc - NIPPON TALC ⁶: glycerol 99.5% - SOLVAY ⁷ : Ucare polymer JR400 LT - AMERCHOL ⁸ : DC 2-1691 émulsion - DOW CORNING | | | | | | |

Les fibres de polymère superabsorbant sont mises à gonfler dans une partie de l'eau de la composition, puis cette dispersion est introduite dans le mélange constitué par le reliquat de la formule, hormis le parfum. Le tout est porté à la température de 60 °C et est soumis à une forte agitation. Après dissolution des composés hydrosolubles et homogénéisation du milieu, on laisse refroidir sous agitation. A 30 °C, on ajoute le parfum, puis on laisse refroidir à température ambiante. On obtient un shampooing d'aspect fibreux qui, appliqué sur cheveux, possède de bonnes propriétés lavantes.

## Revendications

1. Composition de lavage, notamment des matières kératiniques comprenant, dans un milieu physiologiquement acceptable, au moins :
- un polymère superabsorbant présent dans la composition sous forme de fibres, et
- un tensioactif détergent anionique, non ionique ou amphotère.

2. Composition selon la revendication précédente, comprenant de 1 à 50 %, voire de 5 à 25 % en poids de polymère(s) superabsorbant(s) exprimé à l'état sec par rapport à son poids total.

3. Composition selon l'une quelconque des revendications précédentes, dans laquelle les fibres de polymère(s) ont à l'état sec une longueur moyenne en nombre supérieure ou égale à 0,5 mm.

4. Composition selon la revendication précédente, dans laquelle lesdites fibres de polymère(s) possèdent à l'état sec une longueur moyenne en nombre supérieure ou égale à 1 mm, notamment allant de 1 à 100 mm, en particulier de 2 à 80 mm, voire de de 3 à 60 mm, et mieux variant de 3 à 10 mm.

5. Composition selon l'une quelconque des revendications précédentes, dans laquelle ledit polymère superabsorbant est apte, lorsqu'il est à l'état sec, à absorber au moins 20 fois son poids en eau.

6. Composition selon l'une quelconque des revendications précédentes, dans laquelle ledit polymère résulte de la polymérisation avec réticulation partielle de monomères à insaturation éthylénique hydrosolubles, comme les polymères acryliques ou vinyliques.

7. Composition selon la revendication précédente, dans laquelle ledit polymère est un polyacrylate réticulé, partiellement ou totalement neutralisé.

8. Composition selon l'une quelconque des revendications précédentes, comprenant plus de 3 %, notamment de 4 à 50 %, en particulier de 6 à 25 %, voire de 10 à 18 % en poids en tensioactif(s) détergent(s).

9. Composition selon l'une quelconque des revendications précédentes, comprenant au moins un tensioactif anionique.

10. Composition selon la revendication précédente, dans laquelle ledit tensioactif anionique est choisi parmi :
- les sels d'alkylsulfates, d'alkyléthersulfates, d'alkylamidoéthersulfates, d'alkylarylpolyéthersulfates, de monoglycérides sulfates ; d'alkylsulfonates, d'alkylphosphates, d'alkylamidesulfonates d'alkylarylsulfonates, d'α-, oléfine-sulfonates, deparaffine-sulfonates ; d'alkylsulfosuccinates, d'alkyléthersulfosuccinates, d'alkylamidesulfosuccinates; d'alkylsulfosuccinamates ; d'alkylsulfoacétates ; d'alkylétherphosphates; d'acylsarcosinates ; d'acyliséthionates et de N-acyltaurates,
- les sels d'acides gras tels que les sels des acides oléique, ricinoléique, palmitique, stéarique, des acides d'huile de coprah ou d'huile de coprah hydrogénée ; les acyl-lactylates dont le radical acyle comporte 8 à 20 atomes de carbone, et
- les acides d'alkyl D galactoside uroniques et leurs sels ainsi que les acides éthers carboxyliques polyoxyalkylénés et leurs sels, et leurs mélanges.

11. Composition selon la revendication précédente, dans laquelle le tensioactif anionique est choisi parmi le lauryléthersulfate de sodium ou d'ammonium, le laurylsulfate de sodium ou d'ammonium et leurs mélanges.

12. Composition selon l'une quelconque des revendications précédentes, comprenant au moins un tensioactif amphotère.

13. Composition selon la revendication précédente, dans laquelle ledit tensioactif amphotère est choisi parmi les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant.

14. Composition selon la revendication 13, dans laquelle le tensioactif amphotère est choisi parmi les alkyl (C₈-C₂₀) bétaïnes, les sulfobétaïnes, les alkyl (C₈-C₂₀) amidoalkyl (C₁-C₆) betaïnes, les alkyl (C₈-C₂₀) amidoalkyl (C₁-C₆) sulfobétaïnes et leurs mélanges.

15. Composition selon la revendication 13 ou 14, dans laquelle ledit tensioactif amphotère est choisi parmi la cocobétaïne, la cocoamidopropylbétaïne et leurs mélanges.

16. Composition selon l'une quelconque des revendications précédentes, comprenant au moins un tensioactif non ionique.

17. Composition selon la revendication précédente, dans laquelle ledit tensioactif non ionique est choisi parmi les alcools gras polyéthoxylés, polypropoxylés ou polyglycérolés, les alpha-diols gras polyéthoxylés, polypropoxylés ou polyglycérolés, les alkylphénols gras polyéthoxylés, polypropoxylés ou polyglycérolés et les acides gras polyéthoxylés, polypropoxylés ou polyglycérolés, les copolymères d'oxyde d'éthylène et de propylène, les condensats d'oxyde d'éthylène et de propylène sur des alcools gras ; les amides gras polyéthoxylés, les amides gras polyglycérolés ; les esters d'acides gras du sorbitan oxyéthylénés ; les esters d'acides gras du sucrose, les esters d'acides gras du polyéthylèneglycol, les alkylpolyglycosides, les dérivés de N-alkyl glucamine, les oxydes d'amines ; les oxydes de N-acylaminopropylmorpholine et leurs mélanges.

18. Composition selon la revendication précédente, dans laquelle le tensioactif non ionique est choisi parmi les alkylpolyglucosides et leurs mélanges.

19. Composition selon l'une quelconque des revendications précédentes, dont la viscosité est supérieure à 1 Pa.s à 25 °C.

20. Composition selon l'une quelconque des revendications précédentes, comprenant en outre des particules insolubles différentes des fibres de polymère superabsorbant.

21. Composition selon la revendication précédente, comprenant de 0,001 % à 20 % en poids, de préférence de 0,01 % à 10 % en poids et plus particulièrement de 0,1 à 3 % en poids de particules insolubles.

22. Composition selon l'une quelconque des revendications 20 et 21, dans laquelle les particules sont minérales.

23. Composition selon la revendication précédente, dans laquelle les particules minérales comprennent un élément des colonnes IIa, IIIa, IVa, IIIb et IVb du tableau de classification périodique des éléments et de préférence des colonnes IIa, IIIa et IVa.

24. Composition selon la revendication précédente, dans laquelle les particules sont choisies parmi les particules contenant au moins 10 % en poids de carbonate de calcium ou d'au moins un silicate tel que le talc, les particules contenant au moins 90 % d'oxyde d'aluminium, les silices, l'oxyde de magnésium, les oxydes de zinc, les oxydes de titane, le sulfate de baryum, le nitrure de bore et leurs mélanges.

25. Composition selon l'une quelconque des revendications 20 à 24, dans laquelle les particules sont sous forme de plaquettes.

26. Composition selon l'une quelconque des revendications précédentes, associant des fibres de polymère acrylate réticulé à des plaquettes de talc et/ou de nitrure de bore.

27. Composition selon l'une quelconque des revendications précédentes, comprenant de l'eau, notamment plus de 30 %, voire de 50 à 95 % en poids d'eau par rapport au poids total de la composition.

28. Composition selon l'une quelconque des revendications précédentes, sous la forme d'un shampooing.

29. Utilisation d'un polymère superabsorbant sous forme de fibres tel que défini dans l'une quelconque des revendications 1 à 8 pour la préparation d'une composition de lavage des fibres kératiniques, notamment un shampooing.

30. Procédé de traitement cosmétique des matières kératiniques qui comprend l'application d'une quantité efficace d'une composition telle que définie dans l'une quelconque des revendications 1 à 28 sur les matières kératiniques et la réalisation d'un rinçage après un éventuel temps de pose.

## Claims

1. Washing composition, in particular for washing keratinous substances, comprising, in a physiologically acceptable medium, at least:
- one superabsorbent polymer present in the composition in the form of fibres, and
- one anionic, nonionic or amphoteric detergent surfactant.

2. Composition according to the preceding claim, comprising from 1 to 50% by weight, indeed even from 5 to 25% by weight, of superabsorbent polymer(s), expressed in the dry state, with respect to its total weight.

3. Composition according to either one of the preceding claims, in which the polymer fibres have, in the dry state, a number-average length of greater than or equal to 0.5 mm.

4. Composition according to the preceding claim, in which the said polymer fibres have, in the dry state, a number-average length of greater than or equal to 1 mm, in particular ranging from 1 to 100 mm, especially from 2 to 80 mm, indeed even from 3 to 60 mm, and better still varying from 3 to 10 mm.

5. Composition according to any one of the preceding claims, in which the said superabsorbent polymer is capable, when it is in the dry state, of absorbing at least 20 times its weight of water.

6. Composition according to any one of the preceding claims, in which the said polymer results from the polymerization, with partial crosslinking, of watersoluble monomers comprising ethylenic unsaturation, such as acrylic or vinyl polymers.

7. Composition according to the preceding claim, in which the said polymer is a crosslinked and partially or completely neutralized polyacrylate.

8. Composition according to any one of the preceding claims, comprising more than 3% by weight, in particular from 4 to 50% by weight, especially from 6 to 25% by weight, indeed even from 10 to 18% by weight, of detergent surfactant (s).

9. Composition according to any one of the preceding claims, comprising at least one anionic surfactant.

10. Composition according to the preceding claim, in which the said anionic surfactant is chosen from:
- salts of alkyl sulphates, of alkyl ether sulphates, of alkylamido ether sulphates, of alkylaryl polyether sulphates, of monoglyceride sulphates; of alkylsulphonates, of alkyl phosphates, of alkylamidesulphonates, of alkylarylsulphonates, of α-olefinsulphonates, of paraffinsulphonates; of alkyl sulphosuccinates, of alkyl ether sulphosuccinates, of alkylamide sulphosuccinates; of alkyl sulphosuccinamates; of alkyl sulphoacetates; of alkyl ether phosphates; of acylsarcosinates; of acylisethionates and of N-acyltaurates,
- salts of fatty acids, such as salts of oleic acid, ricinoleic acid, palmitic acid, stearic acid, coconut oil acid or hydrogenated coconut oil acid; acyl lactylates, the acyl radical of which comprises from 8 to 20 carbon atoms, and
- alkyl-D-galactosideuronic acids and their salts, and also polyoxyalkylenated ether carboxylic acids and their salts, and their mixtures.

11. Composition according to the preceding claim, in which the anionic surfactant is chosen from sodium lauryl ether sulphate, ammonium lauryl ether sulphate, sodium lauryl sulphate, ammonium lauryl sulphate and their mixtures.

12. Composition according to any one of the preceding claims, comprising at least one amphoteric surfactant.

13. Composition according to the preceding claim, in which the said amphoteric surfactant is chosen from aliphatic secondary or tertiary amine derivatives in which the aliphatic radical is a linear or branched chain comprising from 8 to 18 carbon atoms and comprising at least one water-solubilizing anionic group.

14. Composition according to Claim 13, in which the amphoteric surfactant is chosen from (C₈-C₂₀) alkyl betaines, sulphobetaines, (C₈-C₂₀)alkyl amido(C₁-C₆)alkyl betaines, (C₈-C₂₀) alkyl amido (C₁-C₆) alkyl sulphobetaines and their mixtures.

15. Composition according to Claim 13 or 14, in which the said amphoteric surfactant is chosen from coco betaine, cocoamidopropyl betaine and their mixtures.

16. Composition according to any one of the preceding claims, comprising at least one nonionic surfactant.

17. Composition according to the preceding claim, in which the said nonionic surfactant is chosen from polyethoxylated, polypropoxylated or polyglycerolated fatty alcohols, polyethoxylated, polypropoxylated or polyglycerolated fatty α-diols, polyethoxylated, polypropoxylated or polyglycerolated fatty alkylphenols and polyethoxylated, polypropoxylated or polyglycerolated fatty acids, copolymers of ethylene oxide and of propylene oxide, condensates of ethylene oxide and of propylene oxide with fatty alcohols; polyethoxylated fatty amides, polyglycerolated fatty amides; oxyethylenated sorbitan fatty acid esters; sucrose fatty acid esters, polyethylene glycol fatty acid esters, alkylpolyglycosides, N-alkylglucamine derivatives, amine oxides; N-acylaminopropylmorpholine oxides and their mixtures.

18. Composition according to the preceding claim, in which the nonionic surfactant is chosen from alkylpolyglucosides and their mixtures.

19. Composition according to any one of the preceding claims, the viscosity of which is greater than 1 Pa.s at 25°C.

20. Composition according to any one of the preceding claims, additionally comprising insoluble particles other than the superabsorbent polymer fibres.

21. Composition according to the preceding claim, comprising from 0.001% to 20% by weight, preferably from 0.01% to 10% by weight and more particularly from 0.1 to 3% by weight of insoluble particles.

22. Composition according to either one of Claims 20 and 21, in which the particles are inorganic.

23. Composition according to the preceding claim, in which the inorganic particles comprise an element from Groups IIa, IIIa, IVa, IIIb and IVb of the Periodic Table of the Elements and preferably from Groups IIa, IIIa and IVa.

24. Composition according to the preceding claim, in which the particles are chosen from particles comprising at least 10% by weight of calcium carbonate or of at least one silicate, such as talc, particles comprising at least 90% of aluminium oxide, silicas, magnesium oxide, zinc oxides, titanium oxides, barium sulphate, boron nitride and their mixtures.

25. Composition according to any one of Claims 20 to 24, in which the particles are in the form of platelets.

26. Composition according to any one of the preceding claims, in which crosslinked acrylate polymer fibres are combined with boron nitride and/or talc platelets.

27. Composition according to any one of the preceding claims, comprising water, in particular more than 30% by weight of water, indeed even from 50 to 95% by weight of water, with respect to the total weight of the composition.

28. Composition according to any one of the preceding claims, in the form of a shampoo.

29. Use of a superabsorbent polymer in the form of fibres as defined in any one of Claims 1 to 8 in the preparation of a composition for washing keratinous fibres, in particular a shampoo.

30. Method for the cosmetic treatment of keratinous fibres, which comprises application of an effective amount of a composition as defined in any one of Claims 1 to 28 to the keratinous substances and the performance of a rinsing operation, after an optional leave-in time.

## Patentansprüche

1. Waschzusammensetzung, insbesondere für Keratinsubstanzen, die, in einem physiologisch verträglichen Medium, mindestens folgendes umfasst:
- ein Superabsorptionspolymer, das in der Zusammensetzung in Form von Fasern vorhanden ist, und
- ein anionisches, nicht ionisches oder amphoteres Tensid;

2. Zusammensetzung nach dem vorhergehenden Anspruch, umfassend 1 bis 50 Gew.-%, insbesondere 5 bis 25 Gew.-% Superabsorptionspolymer(e), ausgedrückt im trockenen Zustand, bezogen auf ihr Gesamtgewicht,

3. Zusammensetzung nach einem der vorhergehenden Ansprüche, wobei die Fasern von Polymer(en) im trockenen Zustand eine zahlenmittlere Länge von größer oder gleich 0,5 mm aufweisen.

4. Zusammensetzung nach dem vorhergehenden Anspruch, wobei die Fasern von Polymer(en) im trockenen Zustand eine zahlenmittlere Länge von größer oder gleich 1 mm, insbesondere von 1 bis 100 mm, speziell von 2 bis 80 mm, ganz besonders 3 bis 60 mm, und besser von 3 bis 10 mm variierend aufweist.

5. Zusammensetzung nach einem der vorhergehenden Ansprüche, wobei das Superabsorptionspolymer, wenn es im trockenen Zustand vorliegt, dazu geeignet ist, mindestens das 20-Fache seines Gewichts an Wasser zu absorbieren.

6. Zusammensetzung nach einem der vorhergehenden Ansprüche, wobei das Polymer das Ergebnis der Polymerisation mit teilweiser Vernetzung von wasserlöslichen Monomeren mit ethylenischer Ungesättigtheit, wie acrylische oder vinylische Polymere, ist.

7. Zusammensetzung nach dem vorhergehenden Anspruch, wobei das Polymer ein teilweise oder vollständig neutralisiertes, vernetztes Polyacrylat ist.

8. Zusammensetzung nach einem der vorhergehenden Ansprüche, die mehr als 3 Gew.-%, insbesondere 4 bis 50 Gew.-%, speziell 6 bis 25 Gew.-%, ganz besonders 10 bis 18 Gew.-% grenzflächenaktives Detergens (grenzflächenaktive Detergentien) einschließt.

9. Zusammensetzung nach einem der vorhergehenden Ansprüche, die mindestens ein anionisches Tensid einschließt.

10. Zusammensetzung nach dem vorhergehenden Anspruch, wobei das anionische Tensid ausgewählt ist aus:
- Alkylsufat-, Alkylethersufat-, Alkylamidoethersufat-, Alkylarylpolyethersufat-, Monoglyceridsulfat-, Alkylsulfonat-, Alkylphosphat-, Alkylamidsulfonat-, Alkylarylsulfonat-, α-Olefinsulfonat-, Deparaffinsulfonat-, Alkylsulfosuccinat-, Alkylethersulfosuccinat -, Alkylamidsulfosuccinat-, Alkylsulfasuccinamat-, Alkylsulfoacetat-, Alkyletherphosphat-, Acylsarcosinat-, Acylisethionat und N-Acyltauratsalzen,
- Salzen von Fettsäuren, wie Ölsäure-, Ricinolsäure-, Palmitinsäure-, Stearinsäure-, Säuren von Coprahöl oder hydriertem Coprahöl; Acyllactylaten, deren Acylrest 8 bis 20 Kohlenstoffatome einschließt, und
- Alkyl-D-galaktosiduronsäuren und ihren Salzen sowie polyoxyalkylierten Carboxylsäureethern und ihren Salzen und ihren Gemischen.

11. Zusammensetzung nach dem vorhergehenden Anspruch, wobei das anionische Tensid aus Natrium- oder Ammoniumlaurylethersulfat, Natrium- oder Ammoniumlaurylsulfat und ihren Gemischen ausgewählt ist.

12. Zusammensetzung nach einem der vorhergehenden Ansprüche, die mindestens ein amphoteres Tensid einschließt.

13. Zusammensetzung nach dem vorhergehenden Anspruch, wobei das amphotere Tensid ausgewählt ist aus sekundären oder tertiären aliphatischen Aminderivaten, in denen der aliphatische Rest eine lineare oder verzweigte Kette ist, die 8 bis 18 Kohlenstoffatome einschließt und mindestens eine anionische Hydrosolubilisierungsgruppe enthält.

14. Zusammensetzung nach Anspruch 13, wobei das amphotere Tensid ausgewählt ist aus (C₈-C₂₀)-Alkylbetainen, Sulfobetainen, (C₈-C₂₀)-Alkyl-(C₁-C₆)-amidoalkylbetainen, (C₈-C₂₀)-Alkyl-(C₁-C₆)-amidoalkylbetainen, (C₈-C₂₀)-Alkyl-(C₁-C₆)-amidoalkylsulfobetainen und ihren Gemischen.

15. Zusammensetzung nach Anspruch 13 oder 14, wobei das amphotere Tensid ausgewählt ist aus Cocobetain, Cocoamidopropylbetain und ihren Gemischen.

16. Zusammensetzung nach einem der vorhergehenden Ansprüche, die mindestens ein nicht ionisches Tensid einschließt.

17. Zusammensetzung nach dem vorhergehenden Anspruch, wobei das nichtionische Tensid ausgewählt ist aus polyethxylierten, polypropoxylierten oder polyglycerolierten Fettalkoholen, polyethxylierten, polypropoxylierten oder polyglycerolierten α-Fettdiolen, polyethxylierten, polypropoxylierten oder polyglycerolierten Fettalkylphenolen, und polyethxylierten, polypropoxylierten oder polyglycerolierten Fettsäuren, Ethylen- und Propylenoxid-Copolymeren, Kondensaten von Ethylen- und Propylenoxid mit Fettakoholen; polyethxylierten Fettamiden, polypropoxylierten Fettamiden, polyglycerolierten Fettamiden, Oxyethylensorbiatanfettsäureestern; Saccharosefettsäureestern, Polyethylengylcolfettsäureesstern, Alkylpolyglycosiden, N-Alkylglucaminderivaten, Aminoxiden, N-Acylaminoporpoylmorpholinoxiden und ihren Gemischen.

18. Zusammensetzung nach dem vorhergehenden Anspruch, wobei das nichtionische Tensid ausgewählt ist Alkypolyglucosiden und ihren Gemischen.

19. Zusammensetzung nach einem der vorhergehenden Ansprüche, deren Viskosität größer ist als 1 Pa.s bei 25°C.

20. Zusammensetzung nach einem der vorhergehenden Ansprüche, umfassend weiterhin unlösliche, von superabsorbierenden Polymerfasern verschiedene Teilchen.

21. Zusammensetzung nach dem vorhergehenden Anspruch, umfassend 0,001 bis 20 Gew.-%, vorzugsweise 0,01 bis 10 Gew.-% und ganz besonders 0,1 bis 3 Gew.-% unlösliche Teilchen.

22. Zusammensetzung nach einem der Ansprüche 20 und 21, in denen die Teilchen mineralisch sind.

23. Zusammensetzung nach dem vorhergehenden Anspruch, wobei die mineralischen Teilchen ein Element aus den Reihen IIa, IIIa, IVa, IIIb und IVb des Periodensystems der Elemente und vorzugsweise aus den Reihen IIa, IIIa, IVa, einschließt.

24. Zusammensetzung nach dem vorhergehenden Anspruch, wobei die Teilchen aus den Teilchen ausgewählt sind, die mindestens 10 Gew.-% Calciumcarbonat oder mindestens ein Silikat enthalten, wie Talk, wobei die Teilchen mindestens 90 % Aluminiumoxid enthalten, Kieselsäuren, Magnesiumoxid, Zinkoxiden, Titanoxiden, Bariumsulfat, Bornitrid und ihren Gemischen.

25. Zusammensetzung nach einem der Ansprüche 20 bis 24, wobei die Teilchen in Form von Plättchen vorliegen.

26. Zusammensetzung nach einem der vorhergehenden Ansprüche, die Fasern aus vernetzem Acrylatpolymer mit Plättchen von Talk und/oder Bornitrid kombiniert.

27. Zusammensetzung nach einem der vorhergehenden Ansprüche, die Wasser, insbesondere mehr als 30 Gew.-%, sogar 50 bis 95 Gew.-% Wasser, bezogen auf das Gesamtgewicht der Zusammensetzung einschließt.

28. Zusammensetzung nach einem der vorhergehenden Ansprüche in der Form eines Schampoos.

29. Verwendung von einem superabsorbierenden Polymer in der Form von Fasern, wie in einem der Ansprüche 1 bis 8 definiert, zur Herstellung einer Waschzusammensetzung für Keratinfasern, insbesondere ein Schampoo.

30. Verfahren zur kosmetischen Behandlung der Keratinsubstanzen, das das Aufbringen einer wirksamen Menge einer Zusammensetzung wie in einem der Ansprüche 1 bis 28 definiert, auf die Keratinsubstanzen und die Durchführung einer Spülung nach einer möglichen Einwirkzeit umfasst.
